Europäisches Patentamt

European Patent Office

Office européen des brevets

(19)

(11) Publication number: **0 401 910**

**A1**

(12) **EUROPEAN PATENT APPLICATION**

(21) Application number: **90201399.4**

(22) Date of filing: **29.05.90**

(51) Int. Cl.⁵: **A61F 2/80, A61F 2/60**

(30) Priority: **29.05.89 NL 8901357**

(43) Date of publication of application:
**12.12.90 Bulletin 90/50**

(84) Designated Contracting States:
**AT BE CH DE DK ES FR GB GR IT LI LU NL SE**

(71) Applicant: **Orthopedische Instrumentenmakerij Het Roessingh Roessinghsbleekweg 33**

NL-7522 AH Enschede(NL)

(72) Inventor: **Van de Veen, Paul Gerard Minkmaatstraat 42**
**NL-7514 EC Enschede(NL)**

(74) Representative: **Hoijtink, Reinoud et al OCTROOIBUREAU ARNOLD & SIEDSMA Sweelinckplein 1**
**NL-2517 GK Den Haag(NL)**

(54) **Universal leg prosthesis.**

(57) A universal leg prosthesis comprise a sheath-like body 1 open on one side, a longitudinally adjustable tubular body 3 fixed on the closed side of the sheath-like body 1 and a treading body 4 fixed on the free outer end of the tubular body 3. The edge 2 on the open side of the sheath-like body 1 is bent outward over a portion of the periphery, forming a tuberosity ridge. In order to achieve optimum support for the leg prosthesis, the leg stump 6 is fastened against the side of the sheath-like body 1 lying in the line of the tuberosity ridge by fastening means 5. Thus the loads on the leg prosthesis are directly transmitted to the ischial tuberiosity 19.

FIG.1

## UNIVERSAL LEG PROSTHESIS

The invention relates to a universal leg prosthesis comprising: a longitudinally stiff sheath-like body open on one side, the edge of which on the open side is bent outward over a portion of the periphery; a tubular body adjustable in lengthwise direction fixed on the closed side of the sheath-like body approximately in the line of the longitudinal axis of that body; and a treading body fixed on the free outer end of the tubular body.

For satisfactory rehabilitation of patients who have had a leg amputated it is very important that these patients begin to walk again as soon as possible after the amputation. This increases the chance of retaining or recovering muscle power, mobility and coordination and creates better conditions for rapid healing of the wound and reduction of stump oedema. In addition it is often of great psychological importance for the patient to stand "on his own two feet" again as quickly as possible.

A problem which occurs here, however, is that a leg stump only assumes its definitive form several months after the amputation. Use is therefore generally made of training or interim prostheses in the period between the moment of amputation and the provision of a permanent prosthesis.

There are two sorts of training prostheses, namely the individually manufactured training prosthesis and the training prosthesis for universal use. In the case of an individually manufactured training prosthesis, the portion of the prosthesis in which the leg stump is received is made to size for the patient from a simply deformable material, such as for instance plaster or a plastic. With a universally applicable training prosthesis, the portion of the prosthesis in which the leg stump is received is provided with means whereby this portion can be simply adapted to the dimensions of the leg stumps of the various users.

A great problem with universally applicable training prostheses is usually the fit of the prosthesis. In order to be worn comfortably the leg prosthesis must namely preferably be supported, just as in the case of a leg, on parts of the body which are suitable for withstanding heavy loadings for long periods, such as parts of the skeleton and some tendons. With upper leg prostheses the ischial tuberosity (tuber ischiadicum) is preferably chosen as a suitable body part for the support, whereto most upper leg prostheses are provided at the top with an edge bent over through a part of the periphery, the so-called tuberosity ridge. Lower leg prostheses usually support on the knee cap (patella tendon) or, when the stump is too short to fix the lower leg prosthesis optimally thereon, it is supported by means of a thigh corset on the upper leg, while prostheses for amputations through the knee joint (the so-called knee exarticulations) are usually supported on the extremity of the upper leg.

Until now, however, it has not been found possible to construct universally applicable training prostheses such that for each form of amputation (upper leg amputation, knee ex-articulation and lower leg amputation) an optimal support for the prosthesis on a part of the body suitable therefor is achieved. The universally applicable training prostheses in the known art are constructed such that they support the leg stump clamped over a large portion of its length so that in any case the extremity of the leg stump remains unloaded. The principle of clamping a leg stump along a portion of its length is used, among others, in the frequently used air cushion prosthesis (the so-called pneumatic post amputation mobility aid), the tulip limb and the prosthesis constructed of strip segments. This method for fastening the leg prosthesis has the great drawback however that thereby the loads on the leg prosthesis are in the first instance transmitted into the tissue of the leg stump, and only thereafter passed on to those parts of the body suitable for withstanding high loads for a long period. The tissue is thus unsuitably and disagreeably loaded.

The present invention therefore has for its object to provide a universally applicable leg prosthesis which can be well supported under all conditions because it directly engages the ischial tuberosity, and wherein the heretofore mentioned drawbacks are avoided.

This is achieved according to the invention in that the sheath-like body of the universal leg prosthesis is provided on the inside with means for fastening the leg stump against the side of the body situated in the line of the bent over edge. In a preferred embodiment of the universal leg prosthesis according to the invention, the means for fixing the leg stump consist of straps attached to the inside of the sheath-like body that are continuously adjustable in lengthwise direction and the outer ends of which are situated outside the sheath-like body. These straps are preferably relatively stiff in order to distribute the fixing forces as well as possible over the surface of the leg tissue and to facilitate fitting of the prosthesis. These straps are preferably provided at the ends with Velcro tape. In a preferred embodiment of the invention the treading body is a foot prosthesis. In another preferred embodiment of the universal leg prosthesis according to the invention the sheath-like body is provided with a slit beginning at the open side and running along a large portion of the length of the body, whereby the sheath-like body can be bent open to facilitate putting on and taking off the prosthesis, and with fastening means bridging the slit wherewith the sheath can be closed again after being put on. Provisions are preferably arranged on the sheath-like body for attaching a shoulder strap. The tubular body on the outer end of the prosthesis is preferably continuously adjustable in lengthwise direction.

Mentioned and other features of the universal leg prosthesis according to the invention are described hereinafter in the light of a number of embodiments, to which the invention is not limited, while reference is made to the annexed drawing, in which corresponding numerals designate corresponding components, and:

figure 1 is a view of the prosthesis, a leg stump and a portion of the pelvis of a patient;

figure 2 shows a top view of the prosthesis with the fastening means arranged therein; and

figure 3 shows a partially sectional front view of the mechanism for adjusting the length of the prosthesis.

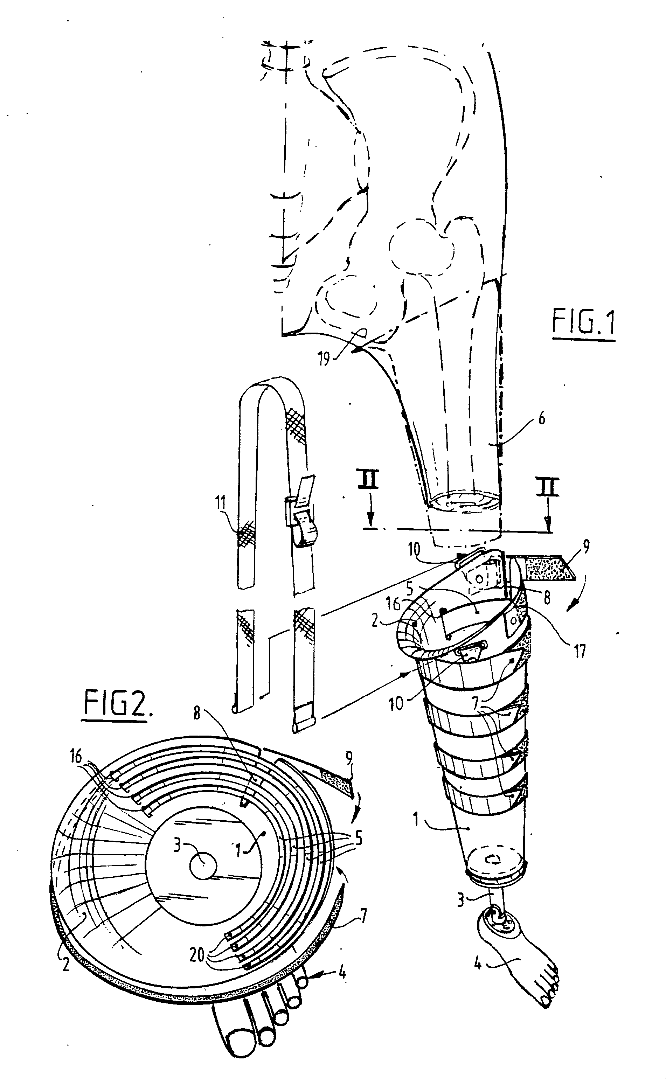

Figure 1 shows a sheath-like body 1 open on one side, of which the edge 2 on the open side is bent outward over a part of the periphery, whereby a so-called tuberosity ridge is formed. The sheath-like body 1 is provided on the closed side with a longitudinally adjustable tubular body 3 attached approximately in the line of the axis of the body, and on the free end of which is fixed a treading body 4. The sheath-like body 1 is provided on the inside with means 5 for fastening a leg stump 6 against the side of the body lying in the line of the tuberosity ridge. These fastening means 5 run from the point where they are fastened to the sheath-like body 1 along a large portion of the peripheral direction of the body and on the interior thereof, to finally leave the body through slots 16. The fastening means 5 are provided with Velcro tape on their free outer ends 7 on the side facing the sheath-like body 1. In addition the fastening means 5 are provided with Velcro tape along a portion of their length lying outside the sheath-like body 1 on the side facing away from the sheath-like body 1. The sheath-like body 1 is provided over a portion of its length with a slit 8 which, when the prosthesis is in use, is closed by fastening means 9 bridging the slit. The fastening means 9 comprise a strap, which on one side of the slit 8 is fixed onto the sheath-like body 1, and the free end of which, which is provided on the side facing the sheath-like body 1 with Velcro tape, can be fastened to a strip of Velcro tape 17 fixed onto the sheath-like body 1.

Before the prosthesis is put on, the outer ends of the fastening means 5 and 9 are loosened, and the sheath like body 1 is bent out slightly whereby step-in is facilitated. The outer ends of the fastening means 5 are then guided into the sheath-like body 1 until the fastening means 5 lie precisely against the inner wall thereof, whereby step-in is further facilitated, and are temporarily fixed in place. The leg stump 6 is positioned in the sheath such that the patient's ischial tuberosity 19 rests precisely on the bent over portion of the edge 2 of the sheath-like body 1, the so-called tuberosity ridge. This optimal position of the prosthesis is indicated in figure 1 by a dashed-dotted line. When the leg stump 6 is positioned in the sheath-like body 1 in this manner the fastening means 5 are tightened and fixed with their outer ends 7 that are provided with Velcro tape. The fastening means 9 are also tightened and fixed. When the patient now walks, the loads on the prosthesis are transmitted directly from the bent over edge 2 of the sheath-like body 1 to the ischial tuberosity 19, and they are distributed therefrom over the patient's skeleton. To further increase the wear comfort of the prosthesis, a shoulder strap 11 can also be attached to fastening rings 10, whereby the user has even better control of the prosthesis.

In figure 2 the sheath-like body 1 is shown with the fastening means 5 arranged therein. The outer ends 20 of the fastening means 5 are fixed on one side of the bent over portion of the edge 2 to the sheath-like body 1, from where the fastening means 5 are guided along the side of the sheath-like body 1 lying opposite the bent over portion of the edge 2 to the slots 16, through which they leave the sheath-like body 1. When a leg stump is placed in the prosthesis and the outer ends 7 of the fastening means 5 are tightened, the parts of the fastening means 5 located inside the sheath-like body 1 will move radially to the side of the sheath-like body situated in the line of the bent over portion of the edge 2, whereby the leg stump 6 is fixed against this side.

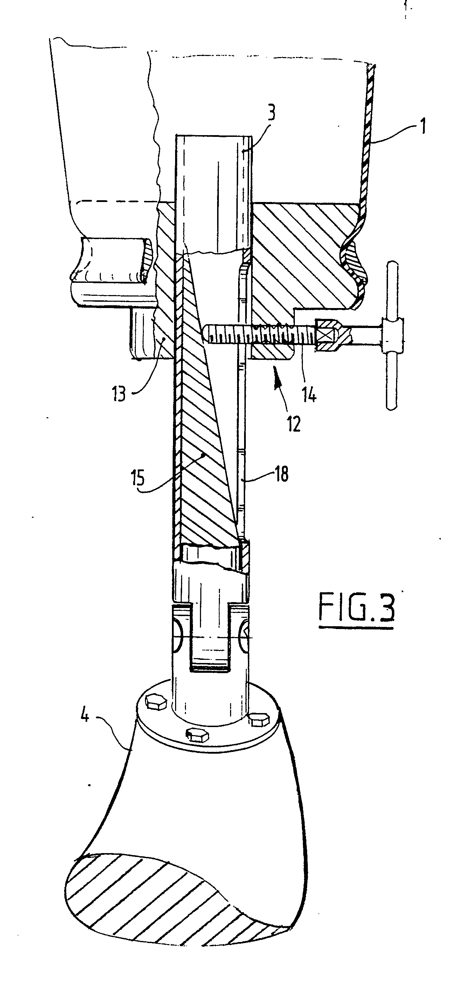

Figure 3 shows the tubular body 3, which is provided with an adjusting mechanism 12, with which the total length of the prosthesis can be varied. The adjusting mechanism 12 comprises a socket 13 mounted on the underside of the sheath-like body 1, in which the tubular body 3 can move up and down, and wherein likewise an adjustable screw bolt 14 is arranged. Through a slot 18 arranged in the wall of the tubular body 3 the screw bolt 14 engages a wedge-shaped part 15, the thickest end part of which is situated on the side of the treading body 4. The length of the prosthesis can be varied by loosening the screw bolt 14, moving the tubular body 3 in the socket 13 upward or downward until the desired position is reached and subsequently tightening the screw bolt 14 again. As a result of the wedge shape of the part 15 of the tubular body 3 widening towards the treading body, the prosthesis will only undergo a slight shortening when loaded in the case the screw bolt 14 unexpectedly become slightly loose.

## Claims

1. Universal leg prosthesis, comprising:
a longitudinally stiff sheath-like body (1) is open on at least one side, whereof the edge (2) on the open side is bent outward over a portion of the periphery;
a tubular body (3) adjustable in lengthwise direction fixed on the closed side of the sheath-like body (1) approximately in the line of the longitudinal axis of that body; and
a treading body (4) fixed on the free outer end of the tubular body (3);
**characterized in that** the sheath-like body (1) is provided on the inside with means (5) for fastening the leg stump (6) against the side of the body situated in the line of the bent over edge (2).

2. Universal leg prosthesis as claimed in claim 1, **characterized in that** the means for fixing the leg stump (6) consists of straps attached to the inside of the sheath-like body (1) that are continuously adjustable in lengthwise direction and run in peripheral direction, the outer ends (7) of which are situated outside the sheath-like body (1).

3. Universal leg prosthesis as claimed in claim 2, characterized in that the straps that are continuously adjustable in lengthwise direction are relatively stiff.

4. Universal leg prosthesis as claimed in claim 3, **characterized in that** the relatively stiff straps that are continuously adjustable in lengthwise direction are provided at the outer ends (7) with Velcro tape.

5. Universal leg prosthesis as claimed in claims 1-4, **characterized in that** the treading body (4) is a foot prosthesis.

6. Universal leg prosthesis as claimed in claims 1-5, **characterized in that** the sheath-like body (1) is provided with a slit (8) beginning at the open side and running along a large portion of the length of the body, and faste ning means (9) bridging the slit.

7. Universal leg prosthesis as claimed in claims 1-6, **characterized in that** the sheath-like body (1) is provided with attachment points (10) for arranging a shoulder strap (11).

8. Universal leg prosthesis as claimed in claims 1-7, **characterized in that** the tubular body (3) is continuously adjustable using an adjusting mechanism (12).

9. Universal leg prosthesis as claimed in claim 8, **characterized in that** the adjusting mechanism (12) is self-braking.

10. Universal leg prosthesis as claimed in claim 9, **characterized in that** the adjusting mechanism (12) comprises a socket (13) that is mounted on the closed side of the sheath-like body (1) and in which the tubular body (3) can move freely in the direction of the longitudinal axis of the sheath-like body (1), and a screw bolt (14) adjustable perpendicularly to this direction for clamping engagement of a part (15) of the tubular body (3).

11. Universal leg prosthesis as claimed in claim 10, **characterized in that** that part (15) of the tubular body (3) displays a wedge-shaped form in lengthwise direction, and that the maximal cross-section is at the end portion directed to the treading body (4).

FIG.1

FIG2.

FIG.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | GB-A- 127 451 (LYNDE) <br> * Entire document * | 1-3,5,6 ,8-10 | A 61 F 2/80 <br> A 61 F 2/60 |
| Y | DE-C- 314 499 (KUNZ) <br> * Figures; page 1, lines 30-43 * | 1-3,5,6 ,8-10 | |
| A | GB-A-2 103 490 (BLATCHFORD) <br> * Page 1, lines 106-114; figures * | 4 | |
| A | FR-A- 482 766 (SVINDT) <br> * Page 2, lines 5-9; figure 5 * | 7 | |
| A | GB-A- 109 085 (MARKS) <br> * Figures; page 3, lines 10-16 * | 8-11 | |
| P,A | US-A-4 842 608 (MARX) <br> * Abstract; figures * | 1 | |

TECHNICAL FIELDS
SEARCHED (Int. Cl.5)

A 61 F

The present search report has been drawn up for all claims

| Place of search | Date of completion of the search | Examiner |
|---|---|---|
| THE HAGUE | 05-09-1990 | STEENBAKKER J. |